Europäisches Patentamt

European Patent Office

Office européen des brevets

(19)

(11) Veröffentlichungsnummer: **0 200 887 B1**

# EUROPÄISCHE PATENTSCHRIFT

(45) Veröffentlichungstag der Patentschrift: **24.07.91**   (51) Int. Cl.⁵: **A61M 16/01**

(21) Anmeldenummer: **86103364.5**

(22) Anmeldetag: **13.03.86**

(54) Umfüllverschluss für chemisch-pharmazeutische Substanzen und Verfahren zu seiner Herstellung.

(30) Priorität: **21.03.85 DE 3510166**

(43) Veröffentlichungstag der Anmeldung:
**17.12.86 Patentblatt 86/46**

(45) Bekanntmachung des Hinweises auf die
Patenterteilung:
**24.07.91 Patentblatt 91/30**

(84) Benannte Vertragsstaaten:
**AT BE CH FR GB IT LI LU NL SE**

(56) Entgegenhaltungen:
**AU-A- 155 298
DE-A- 1 900 271
FR-A- 2 137 642
US-A- 2 847 042
US-A- 3 536 108**

(73) Patentinhaber: **KLAUS F. MÜLLER PHARMA-
VERKAUFSBÜRO
Am Siebenstein 3
W-6072 Dreieich(DE)**

(72) Erfinder: **Müller, Klaus F.
Am Siebenstein 3
W-6072 Dreieich(DE)**

(74) Vertreter: **Eyer, Eckhardt Philipp, Dipl.-Ing. et
al
Patentanwälte Eyer & Linser Robert-
Bosch-Strasse 12a
W-6072 Dreieich(DE)**

**Beschreibung**

Die Erfindung betrifft einen Umfüllverschluß für chemisch-pharmazeutische Substanzen, insbesondere Narkoseflüssigkeiten, Narkosegase oder dergl., aus einer Vershlußkappe zum Aufsetzen auf die Öffnung des die Substanz abgebenden Behälters sowie einem Adapterstück mit Schlüsselmarkierungen zur Herstellung einer Verbindung mit einer entsprechende Gegenmarken aufweisenden Aufnahme des zu füllenden Behälters wobei die Verschlußkappe und der Adapter mittels eines Flüssigkeitsschlauches und eines Lüftungsschlauches miteinander verbunden sind, die in jeweils einer quer verlaufenden Flüssigkeitsbohrung bezw. Lüftungsbohrung münden (siehe DE-A-1900271).

Bei einem bekannten Umfüllverschluß dieser Art sind die Verschlußkappe und das Adapterstück jeweils mit einem Rohrnippel zum Aufschieben eines Schlauchstückes versehen, das auf den Rohrnippeln mittels einer Schelle, im allgemeinen einer Klemmschelle gegen Abziehen gesichert ist. Hierbei ist das Adapterstück mit einer Sacklochbohrung und mit zwei in die Sacklochbohrung einragenden Querbohrungen unterschiedlichen Durchmessers versehen, von denen die Bohrung größeren Querschnittes als Flüssigkeitsbohrung und die Bohrung kleineren Querschnittes zur Halterung des Entlüftungsschlauches dient, der sich durch den gesamten Umfüllverschluß erstreckt und unter Umführung in die Querbohrung gezogen und in dieser gehalten ist. Der bekannte Umfüllverschluß ist in herstellungs- und montagetechnischer Hinsicht außerordentlich aufwendig. Er besteht aus mindestens sechs - zum Teil metallischen zum Teil thermoplastischen - Teilen und erfordert einen erheblichen Montageaufwand insofern, als das Aufschieben der Schlauchenden auf die Rohrnippel, die Aufbringung der Klemmschellen und das Einfädeln des Lüftungsschlauches in gesonderten arbeitsaufwendigen Arbeitsgängen durchgeführt werden müssen, von denen insbesondere das Einfädeln des Lüftungsschlauches wegen der Umführung um 90° innerhalb der engen Flüssigkeitsbohrung besondere Probleme aufwirft.

Der vorliegenden Erfindung liegt als Aufgabe die Schaffung eines eine einfache Herstellung und Montage ermöglichenden Umfüllverschlusses der beschriebenen Art zugrunde. Die Erfindung besteht darin, daß das Adapterstück, der Flüssigkeitsschlauch und die Verschlußkappe aus einem Stück bestehen und eine durchgehende Längsbohrung aufweisen, deren adapterseitige Öffnung mittels eines mit einer Sacklochbohrung versehenen Verschlußstopfens verschlossen ist, in dem der in die Sacklochbohrung einragende Lüftungsschlauch gehalten ist, der mittels einer sich durch das Adapterstück, den Verschlußstopfen sowie die Wandung des Lüftungsschlauches er streckenden Querbohrung mit der Atmosphäre verbunden ist.

Durch die Erfindung ist ein einfach herstellbarer und montierbarer Umfüllverschluß geschaffen, der aus lediglich drei Teilen besteht, die alle aus thermoplastischem Material vorgefertigt und durch einfache Steck-bezw. Klebeverbindung zusammengefügt und durch einen in einem einzigen Arbeitsgang durchführbares Einbringen zweier Querbohrungen zum fertigen Umfüllverschluß kompletiert werden können. Die material- und arbeitsaufwendigen Arbeiten im Zusammenhang mit der Aufbringung der (Klemm)-Schellen und dem Einfädeln des Lüftungsschlauches entfallen.

In einer weiteren Ausführungsform der Erfindung ist zweckmäßig der Flüssigkeitsschlauch mit einer Wellung versehen, wodurch eine große Flexibilität des Umfüllverschlusses erreicht wird, derart, daß auch in einem großen Winkel zum füssigkeitsabgebenden Behälter stehende Gefäße befüllt werden können.

Die Herstellung des Umfüllverschlusses erfolgt erfindungsgemäß in der Weise, daß das Adapterstück, der Flüssigkeitsschlauch und die Verschlußkappe aus einem Stück mit durchgehender Längsbohrung hergestellt, der Lüftungsschlauch in die Sacklochbohrung des Verschlußstopfens eingebracht und der Verschlußstopfen derart in die Längsbohrung eingebracht wird, daß der Lüftungsschlauch innerhalb des Flüssigkeitsschlauches den Umfüllverschluß durchragt, worauf in das Adapterstück zwei Querbohrungen eingebracht werden, von denen die eine als Flüssigkeitsbohrung außerhalb des Verschlußstopfens bis in die Längsbohrung und die andere als Lüftungsbohrung im Bereich des Verschlußstopfens bis in den Lüftungsschlauch geführt ist.

Die Erfindung ist in der einen Längsschnitt durch einen Umfüllverschluß wiedergebenden Zeichnung beispielsweise veranschaulicht.

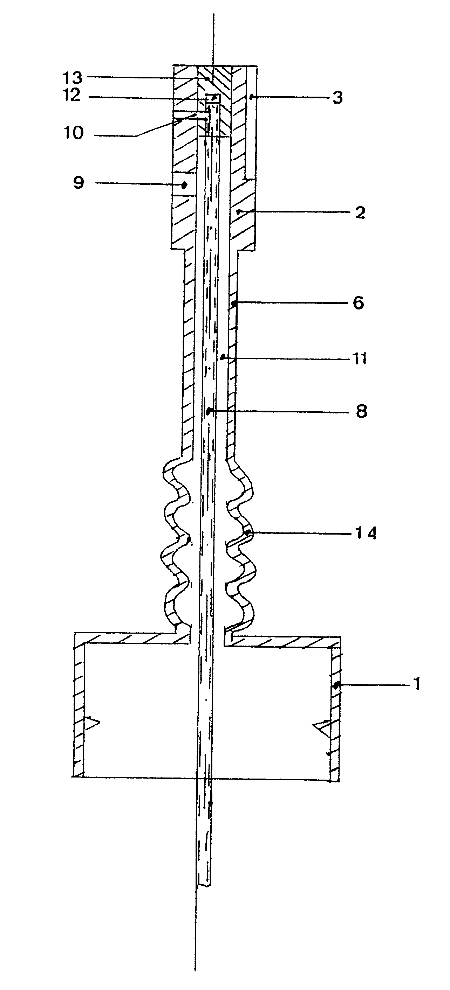

Der in der Zeichnung dargestellte Umfüllverschluß für chemisch-pharmazeutische Substanzen, insbesondere Narkoseflüssigkeiten, Narkosegase oder dergl., besteht aus einer Verschlußkappe 1 zum Aufsetzen auf die Öffnung des die Substanz abgebenden Behälters sowie einem Adapterstück 2 mit einer Schlüsselmarkierung 3 zur Herstellung einer Verbindung mit einer entsprechende Gegenmarken aufweisenden Aufnahme des zu füllenden Behälters. Die Verschlußkappe 1 und das Adapterstück 2 sind mittels eines Flüssigkeitsschlauches 6 und eines sich durch den gesamten Umfüllverschluß erstreckenden, nach der Montage in den Flüssigkeitsbehälter ragenden Lüftungsschlauches 8 miteinander verbunden. Das Adapterstück ist mit jeweils einer quer verlaufenden Flüssigkeitsbohrung 9 bezw. Lüftungsbohrung 10 versehen. Das Adapterstück 3, der Flüssigkeitsschlauch 6 und die Verschlußkappe 1 bestehen aus einem Stück und weisen eine durchgehende Längsbohrung 11 auf, deren adapterseitige Öffnung mittels eines mit einer Sacklochbohrung 12 versehenen Verschlußstopfens 13 verschlossen ist, in dem der in die Sacklochbohrung 12 einragende Lüftungsschlauch 8 gehalten ist. Der Lüftungsschlauch 8 ist mittels einer sich durch das Adapterstück 3, den Verschlußstopfen 13 sowie die Wandung des Lüftungsschlauches 8 erstreckenden Querbohrung 7 mit der Atmosphäre verbunden. Der Flüssigkeitsschlauch 6 ist mit einer Wellung 14 versehen, durch die der Verschluß eine wesentlich verbesserte Flexibilität erhält.

Die Herstellung des Umfüllverschlusses erfolgt erfindungsgemäß in der Weise, daß das Adapterstück 3, der Flüssigkeitsschlauch 6 und die Verschlußkappe 1 aus einem Stück mit durchgehender Längsbohrung 11 hergestellt, der Lüftungsschlauch 8 in die Sacklochbohrung 12 des Verschlußstopfens 13 eingebracht und der Verschlußstopfen 13 derart in die Längsbohrung 11 eingebracht wird, daß der Lüftungsschlauch 8 innerhalb des Flüssigkeitsschlauches 6 den Umfüllverschluß durchragt, worauf in das Adapterstück 3 zwei Querbohrungen 9, 10 eingebracht werden, von denen die eine als Flüssigkeitsbohrung 9 außerhalb des Verschlußstopfens 13 bis in die Längsbohrung 11 und die andere als Lüftungsbohrung 10 im Bereich des Verschlußstopfens 13 bis in den Lüftungsschlauch 8 geführt ist.

## Patentansprüche

1. Umfüllverschluß für chemisch-pharmazeutische Substanzen, insbesondere Narkoseflüssigkeiten, Narkosegase oder dergl., bestehend aus einer Verschlußkappe (1) zum Aufsetzen auf die Öffnung des die Substanz abgebenden Behälters sowie einem Adapterstück (3) mit Schlüsselmarkierungen zur Herstellung einer Verbindung mit einer entsprechende Gegenmarken aufweisenden Aufnahme des zu füllenden Behälters wobei die Verschlußkappe (1) und der Adapter (3) mittels eines Flüssigkeitsschlauches (6) und eines Lüftungsschlauches (8) miteinander verbunden sind, die in jeweils einer quer verlaufenden Flüssigkeitsbohrung (9) bzw. Lüftungsbohrung (10) münden, dadurch gekennzeichnet, daß das Adapterstück (3), der Flüssigkeitsschlauch (6) und die Verschlußkappe (1) aus einem Stück bestehen und eine durchgehende Längsbohrung (11) aufweisen, deren adapterseitige Öffnung mittels eines mit einer Sacklochbohrung (12) versehenen Verschlußstopfens (13) verschlossen ist, in dem der in die Sacklochbohrung (12) einragende Lüftungsschlauch (8) gehalten ist, der mittels einer sich durch das Adapterstück (3), den Verschlußstopfen (13) sowie die Wandung des Lüftungsschlauches (8) erstreckenden Querbohrung (7) mit der Atmosphäre verbunden ist.

2. Umfüllverschluß nach Anspruch 1, dadurch gekennzeichnet, daß der Flüssigkeitsschlauch (6) mit einer Wellung (14) versehen ist.

3. Verfahren zur Herstellung eines Umfüllverschlusses für chemisch-pharmazeutische Substanzen, insbesondere Narkoseflüssigkeiten, Narkosegase oder dergl., nach Anspruch 1 oder 2, dadurch gekennzeichnet, daß das Adapterstück (3), der Flüssigkeitsschlauch (6) und die Verschlußkappe (1) aus einem Stück mit durchgehender Längsbohrung (11) hergestellt, der Lüftungsschlauch (8) in die Sacklochbohrung (20) des Verschlußstopfens (13) eingebracht und der Verschlußstopfen (13) derart in die Längsbohrung (11) eingebracht wird, daß der Lüftungsschlauch (8) innerhalb des Flüssigkeitsschlauches (6) den Umfüllverschluß durchragt, worauf in das Adapterstück (3) zwei Querbohrungen (9, 10) eingebracht werden, von denen die eine als Flüssigkeitsbohrung (9) außerhalb des Verschlußstopfens (13) bis in die Längsbohrung (11) und die andere als Lüftungsbohrung (10) im Bereich des Verschlußstopfens (13) bis in den Lüftungsschlauch (8) geführt ist.

## Claims

1. A filling closure for chemical/pharmaceutical substances, particularly narcotic liquids, narcotic gases or the like, comprising a closure cap (1) for setting on the opening of the container delivering the substance, and an adapter (3) with key markings for producing a connection with a receiving portion, having corresponding key markings, of the container to be filled, the closure cap (1) and the adapter (3) being connected together by a liquid tube (6) and a ventilation tube (8), which open into a transversely-extending liquid bore (9) and ventilation bore (10) respectively, characterised in that the adapter (3), the liquid tube (6) and the closure cap (1) comprises one piece and have a continuous longitudinal bore (11), whose opening at the adapter end is closed by a closure stopper (13) provided with a blind bore (12), the ventilation tube (8) projecting into the blind bore (12) being held in said closure stopper (13), and said ventilation tube (8) being connected to the atmosphere by means of a transverse bore (7) extending through the adapter (3), the closure stopper (13) and the wall of the ventilation tube (8).

2. A filling closure according to Claim 1, characterised in that the liquid tube (6) is provided with a corrugation (14).

3. A method of manufacturing a filling closure for chemical/pharmaceutical substances, particularly narcotic liquids, narcotic gases or the like, according to Claim 1 or 2, characterised in that the adapter (3), the liquid tube (6) and the closure cap (1) are made from one piece with a continuous longitudinal bore (11), the ventilation tube (8) is passed into the blind bore (20) of the closure stopper (13), and the closure stopper (13) is passed into the longitudinal bore (11) in such a way that the ventilation tube (8) projects, inside the liquid tube (6), through the filling closure, whereupon there are made in the adapter (3) two transverse bores (9, 10), of which one, as a liquid bore (9), is passed outside the closure stopper (13) until it extends into the longitudinal bore (11), and the other, as a ventilation bore (10), is passed in the vicinity of the closure stopper (13) until it extends into the ventilation tube (8).

**Revendications**

1. Fermeture de transvasement pour des substances chimico-pharmaceutiques, en particulier pour des anesthésiques liquides, des anesthésiques gazeux ou similaires, se composant d'un capuchon de fermeture (1) destiné à être placé sur l'ouverture du recipient qui délivre la substance, ainsi que d'une pièce de raccord (3) portant des repères codés pour l'établissement d'une communication avec un logement du récipient à remplir, portant des contre-repères correspondants, le capuchon de fermeture (1) et le raccord (3) étant reliés l'un à l'autre au moyen d'un tuyau flexible de liquide (6) et d'un tuyau flexible d'aération (8) qui débouchent respectivement dans une forure de liquide (9) et une forure d'aération (10) dirigées transversalement, caractérisée en ce la pièce de raccord (3), le tuyau flexible de liquide (6) et le capuchon de fermeture (1) sont faits d'une seule pièce et présentent une forure longitudinale continue (11) dont l'orifice du côté raccord est obturé au moyen d'un bouchon de fermeture (13) qui est muni d'un logement à fond plein (12) et dans lequel est maintenu le tuyau flexible d'aération (8) qui est inséré dans le logement à fond plein (12) et qui est en mis communication avec l'atmosphère au moyen d'une forure transversale (7) s'étendant à travers la pièce de raccord (3), à travers le bouchon de fermeture (13) ainsi qu'à travers la paroi du tuyau flexible d'aération (8).

2. Fermeture de transvasement selon la revendication 1, caractérisée en ce que le tuyau flexible de liquide (6) est muni d'une ondulation (14).

3. Procédé de fabrication d'une fermeture de transvasement pour des substances chimico-pharmaceutiques, en particulier pour des anesthésiques liquides, des anesthésiques gazeux ou similaires, selon la revendication 1 ou 2, caractérisé en ce que la pièce de raccord (3), le tuyau flexible de liquide (6) et le capuchon de fermeture (1) sont fabriqués d'une seule pièce avec une forure longitudinale continue (11), le tuyau flexible d'aération (8) est inséré dans le logement à fond plein (12) du bouchon de fermeture (13) et ce dernier est inséré dans la forure longitudinale 11 de telle sorte que le tuyau flexible d'aération (8) traverse la fermeture de transvasement à l'intérieur du tuyau flexible de liquide (6), après quoi il est forme, dans la pièce de raccord (3), deux forures transversales (9, 10) dont l'une, destinée à servir de forure de liquide (9), aboutit dans la forure longitudinale (11) en dehors du bouchon de fermeture (13) et l'autre, destinée à servir de forure d'aération (10), aboutit dans le tuyau flexible d'aération (8) au niveau du bouchon de fermeture (13).